# EUROPEAN PATENT APPLICATION

(11) **EP 4 653 538 A1**
(43) Date of publication of application: **26.11.2025**
(21) Application number: 24744633.9
(22) Date of filing: 16.01.2024
(51) Int. Cl.: C12N 15/87, C12N 15/86, C12N 15/88, C12N 15/89, C12P 21/00, C12N 5/10

(54) **METHOD FOR PRODUCING NUCLEIC ACID INTRODUCED CELLS, AND METHOD FOR PRODUCING USEFUL SUBSTANCE**

(30) Priority: 17.01.2023 JP 2023004817
(71) Applicant: FUJIFILM Corporation, Tokyo 106-8620 (JP)
(72) Inventor: MORI, Yusuke, Ashigarakami-gun, Kanagawa 258-8577 (JP); NISHINO, Masafumi, Ashigarakami-gun, Kanagawa 258-8577 (JP); EDO, Michiko, Ashigarakami-gun, Kanagawa 258-8577 (JP); MURAGUCHI, Taichi, Ashigarakami-gun, Kanagawa 258-8577 (JP)
(74) Representative: Hoffmann Eitle
(86) International application number: PCT/JP2024/000886
(87) International publication number: WO 2024/154711

(57) **Abstract**

An object of the present invention is to provide a method for producing a nucleic acid-introduced cell, in which a transfection efficiency is improved, and to provide a method for producing a useful substance using the nucleic acid-introduced cell. According to the present invention, there is provided a method for producing a nucleic acid-introduced cell, including (a) a treatment step of reducing an unnecessary nucleic acid in a cell-containing solution, and (b) an introduction step of introducing a target nucleic acid into a cell by adding the target nucleic acid to the cell-containing solution.

## Description

### Technical Field

The present invention relates to a method for producing a nucleic acid-introduced cell, in which a transfection efficiency is improved by reducing an amount of an unnecessary nucleic acid present in a cell culture solution. The present invention further relates to a method for producing a useful substance using the nucleic acid-introduced cell.

### Background Art

The approach of transiently expressing exogenous nucleic acid (plasmid or the like) in animal cells is effective as a means for large-scale and rapid production of a recombinant protein. However, since the transient transfection efficiency of the plasmid is low, a large amount of purified DNA is required for mass production of the recombinant protein, which is one of the factors that increase the cost. It is known that the fact that the transfection efficiency of the exogenous nucleic acid into the cell is decreased is that the efficiency is decreased due to the mixing of the E. coli-derived RNA in the purified plasmid. It is common to use an RNase in a case of purifying a plasmid from Escherichia coli, and a purified plasmid from which an RNase has been removed is generally used as an exogenous nucleic acid in transfection. In addition, Patent Document 1 describes that an RNase is used to remove E. coli-derived RNA in a case of purifying a plasmid from E. coli, and the purified plasmid is transfected into cultured cells without removing the RNase. In Patent Document 1, it is inferred that possibility the RNase is inactivated in a case where a plasmid is transfected into cultured cells is high, and the RNA in the cell culture solution is not reduced.

### Prior Art Documents

### Patent Documents

Patent Document 1: WO2002/026966A

### SUMMARY OF THE INVENTION

### Object to be solved by the invention

It is known that the efficiency is decreased due to the contamination of the purified plasmid with the Escherichia coli-derived RNA as described above. On the other hand, it is not known that RNA derived from animal cells inhibits transfection during the culture of animal cells. An object to be solved of the present invention is to provide a method for producing a nucleic acid-introduced cell, in which a transfection efficiency is improved. Another object to be solved of the present invention is to provide a method for producing a useful substance using the above-described nucleic acid-introduced cell.

### Means for solving the object

As a result of intensive studies to achieve the above object, the present inventors have found that cell debris affects the decrease in transfection efficiency and the transfection efficiency is improved by reducing the amount of the unnecessary nucleic acid in the cell-containing solution. The present invention has been completed based on the above findings.

According to the present invention, the following inventions are provided.
<1> A method for producing a nucleic acid-introduced cell, comprising:
   (a) a treatment step of reducing an unnecessary nucleic acid in a cell-containing solution; and
   (b) an introduction step of introducing a target nucleic acid into a cell by adding the target nucleic acid to the cell-containing solution.
<2> The method according to <1>, in which the unnecessary nucleic acid is RNA.
<3> The method according to <1> or <2>, in which the treatment step includes adding an RNase to the cell-containing solution.
<4> The method according to <1> or <2>, in which in the step (a), an amount of the unnecessary nucleic acid is monitored and reduced to an amount equal to or less than a reference value.
<5> The method according to <1> or <2>, in which the target nucleic acid is a purified nucleic acid.
<6> The method according to <1> or <2>, in which the step (b) is performed after the step (a) is performed.
<7> The method according to <1> or <2>, in which the target nucleic acid is DNA.
<8> The method according to <1> or <2>, in which the target nucleic acid is a plasmid.
<9> The method according to <1> or <2>, in which, in the step (b), the target nucleic acid is introduced into the cell by calcium phosphate, DEAE-dextran, a cationic lipid, a polymer, a virus, magnetofection, microinjection, electroporation, or a gene gun.
<10> The method according to <1> or <2>, in which, in the step (b), the target nucleic acid is introduced into the cell by a polymer.
<11> The method according to <10>, in which the polymer is polyethyleneimine.
<12> The method according to <1> or <2>, in which the cell is an HEK cell, a CHO cell, or a Vero cell.
<13> The method according to <1> or <2>, in which a cell density of the cell-containing solution in the step (b) is 1 × 10⁶ cells/mL or more.
<14> The method according to <1> or <2>, in which a volume of the cell-containing solution is 1 L or more.
<15> A method for producing a useful substance, comprising:
   a step of producing a nucleic acid-introduced cell by the method according to any one of <1> to <14>; and
   a step of culturing the nucleic acid-introduced cell.
<16> The method according to <15>, in which the useful substance is a virus vector or a protein.

### Effect of the invention

According to the present invention, it is possible to improve the efficiency (transfection efficiency) of incorporating the exogenous nucleic acid into the cultured cell. According to the present invention, it is possible to improve the production efficiency of a useful substance such as a biopharmaceutical or a reagent.

### BRIEF DESCRIPTION OF THE DRAWINGS

[Fig. 1] Fig. 1 shows an example of a configuration of a cell culture device.
[Fig. 2]Fig. 2 shows a fluorescence image of GFP-expressing cells.
[Fig. 3]Fig. 3 shows the results of detecting the nucleic acid contained in the culture medium by agarose gel electrophoresis.
[Fig. 4]Fig. 4 shows a fluorescence image of GFP-expressing cells.
[Fig. 5]Fig. 5 shows quantification of GFP-expressing positive cells by flow cytometry.
[Fig. 6]Fig. 6 shows the detection of expression of a virus gene by Western blot.

### EMBODIMENTS FOR CARRYING OUT THE INVENTION

Hereinafter, an example of the embodiment of the present disclosure will be described. However, the present disclosure is not limited to the following embodiments, and can be implemented with appropriate changes within the scope of the object of the present disclosure. In the present specification, a numerical range indicated using "to" means a range including numerical values described before and after "to" as a minimum value and a maximum value, respectively.

The present invention relates to a method for producing a nucleic acid-introduced cell, including (a) a treatment step of reducing an unnecessary nucleic acid in a cell-containing solution, and (b) an introduction step of introducing a target nucleic acid into a cell by adding the target nucleic acid to the cell-containing solution. The method for producing a nucleic acid-introduced cell according to the embodiment of the present invention can be used for producing a biopharmaceutical (for example, a virus vector and an antibody) and a reagent, which are produced by introducing an exogenous nucleic acid into a cultured cell.

The step (a) is a treatment step of reducing the unnecessary nucleic acid in the cell-containing solution.

The unnecessary nucleic acid in the cell-containing solution is preferably a nucleic acid derived from the above-described cell. The unnecessary nucleic acid may be RNA or DNA, but is preferably RNA.

As a method of reducing the unnecessary nucleic acid in the cell-containing solution, there are a method of decomposing the unnecessary nucleic acid and a method of adsorbing the unnecessary nucleic acid. Examples of the method of decomposing the unnecessary nucleic acid include a method of digesting the unnecessary nucleic acid with an enzyme.

In a case where the unnecessary nucleic acid is RNA, the treatment step of reducing the unnecessary nucleic acid preferably includes adding an RNase (more preferably RNase A) to the cell-containing solution. In a case where the unnecessary nucleic acid is RNA, the target nucleic acid is preferably a nucleic acid (for example, DNA) other than RNA.

The treatment concentration of the RNase (preferably, RNase A) in the cell-containing solution is preferably 0.1 µg/mL or more, more preferably 1 µg/mL or more, and still more preferably 10 µg/mL or more.

In a case where the unnecessary nucleic acid is DNA, the treatment step of reducing the unnecessary nucleic acid preferably includes adding a DNase to the cell-containing solution. It is noted that in a case where the unnecessary nucleic acid is DNA, the target nucleic acid is preferably a nucleic acid (for example, RNA) other than DNA.

The timing of performing the step (a) is not particularly limited, and the step (a) and the step (b) may be performed at the same time, but it is preferable to perform the step (b) after the step (a) is performed. That is, it is preferable to perform the step (a) before the step (b) is performed.

In a case where the step (a) is performed by an enzyme treatment, the time of the enzyme treatment is not particularly limited as long as the unnecessary nucleic acid can be reduced, but is preferably 1 hour or more, more preferably 2 hours or more, and still more preferably 4 hours or more.

In a case where the type of the unnecessary nucleic acid is the same as the type of the target nucleic acid (that is, in a case where the unnecessary nucleic acid is RNA and the target nucleic acid is RNA, or in a case where the unnecessary nucleic acid is DNA and the target nucleic acid is DNA), it is preferable to perform the introduction step of adding the target nucleic acid to the cell-containing solution to introduce the target nucleic acid into the cell after performing the treatment step of reducing the unnecessary nucleic acid in the cell-containing solution and then removing or inactivating the RNase or DNase used in the treatment step.

In the step (a), the degree of reduction in the unnecessary nucleic acid is preferably such that the unnecessary nucleic acid is reduced to a degree that does not affect the efficiency of the nucleic acid introduction step.

The amount of the unnecessary nucleic acid is reduced to preferably 200% by mass or less and more preferably 100% by mass or less with respect to the amount of the target nucleic acid to be added.

It is preferable that in the step (a), an amount of the unnecessary nucleic acid is monitored and reduced to an amount equal to or less than a reference value. The reference value can be appropriately set, but in a case where the unnecessary nucleic acid is RNA, for example, the value can be set to 3 µg/mL or less, preferably 2 µg/mL or less, more preferably 0.1 µg/mL or less, and still more preferably 0.01 µg/mL or less.

The step (b) is an introduction step of introducing a target nucleic acid into a cell by adding the target nucleic acid to the cell-containing solution.

The nucleic acid refers to a chain-like polynucleotide in which a nucleotide consisting of a purine or pyrimidine base, a sugar, and a phosphate is a basic unit, and the phosphate forms a diester bond between the 3' and 5' carbon atoms of the sugar between each of the nucleotides to polymerize. Due to the difference in sugar, nucleic acids are roughly classified into deoxyribonucleic acid (DNA) having a sugar moiety of deoxyribose and ribonucleic acid (RNA) having a sugar moiety of ribose. The nucleic acid may be any one of DNA (linear DNA or circular DNA) or RNA, and may be any one of an oligonucleotide or a polynucleotide. In the present specification, the nucleic acid is used interchangeably with a gene, DNA, RNA, an oligonucleotide, and a polynucleotide.

The target nucleic acid is preferably DNA. An example of the target nucleic acid is a plasmid.

The target nucleic acid is preferably a purified nucleic acid. The purified nucleic acid is preferably a nucleic acid substantially not containing RNase. The purified nucleic acid is preferably a nucleic acid that does not substantially contain a protein.

As the target nucleic acid, a nucleic acid (gene) for producing a virus as a useful substance or a nucleic acid encoding a protein as a useful substance is used.

The virus means a virus that is produced by introducing a nucleic acid into a cell. Examples of the virus include an adeno-associated virus, a lentivirus, a baculovirus, and a retrovirus. In a case where a virus is produced using RNase for removing unnecessary nucleic acids, a DNA virus that encompasses DNA in the capsid is preferable, and an adeno-associated virus is particularly preferable.

Adeno-associated virus (AAV) refers to a small, incompletely replicative, non-enveloped virus containing single-stranded DNA having about 4,700 bases, from the family of Parvoviridae and Dependoparvovirus. It is known that there are more than 100 serum types in AAV, and that the host range and the characteristics of the virus differ depending on the difference in the serum types. Serum type 2 (AAV2) is one of the serum types that have been widely studied for a long time, and it is known that the host range is very wide. The serum type 1 (AAV1), the serum type 5 (AAV5), the serum type 6 (AAV6), the serum type 8 (AAV8), and the serum type 9 (AAV9) are serum types having higher tissue directivity. It is said that AAV1 has a high gene introduction efficiency into muscle, liver, airway, a central nervous system, and the like, AAV5 has a high gene introduction efficiency into a central nervous system, liver, retina, and the like, AAV6 has a high gene introduction efficiency into heart, muscle, liver, and the like, AAV8 has a high gene introduction efficiency into liver, muscle, a central nervous system, retina, fat, and the like, and AAV9 has a high gene introduction efficiency into heart, a central nervous system, lung, muscle, liver, fat, and the like. In the present invention, the serum type 2 or the serum type 5 is preferably used. The serum type 5 is particularly preferable.

The adeno-associated virus gene refers to a gene composed of one or a plurality of nucleic acid sequences derived from the serum types of one or a plurality of adeno-associated viruses. The adeno-associated virus gene is preferably a gene involved in replication and packaging of AAV and a gene encoding an AAV constituent protein.

AAV is a non-enveloped virus that proliferates in the presence of helper viruses such as adenovirus and herpesvirus. In the preparation of AAV to be used for gene therapy or nucleic acid introduction, classically, AAV replication has been performed by co-infecting host cells with an adenovirus. In addition, a gene responsible for the helper action of adenovirus has been clarified, and a plasmid bearing this gene has also been used. For example, a plasmid containing a Rep gene and a Cap gene, an adenovirus helper plasmid, and a plasmid containing a gene for treatment or prevention are simultaneously transfected into cells, and can thus be packaged as recombinant AAV (rAAV).

The Rep gene and the Cap gene encode proteins involved in the replication and packaging of the virion. In the wild type, the Rep gene is expressed from the p5 promoter and the p19 promoter. The Cap region expresses VP1, VP2, and VP3. Examples of the promoter that is naturally carried by the Cap gene can include a p40 promoter.

The adeno-associated virus gene (such as a Rep gene and a Cap gene) may be a wild-type gene, but a gene in which modifications such as substitution, deletion, insertion, or addition of a base have been made to the wild-type gene may be used as long as it exhibits an inherent function.

In a case where the Rep gene and the Cap gene are introduced into a cell, a vector containing the Rep gene and the Cap gene can be introduced into the cell. The Rep gene means a region of the AAV genome encoding a virus replication protein that is known to those skilled in the art and that is collectively required for replication of a virus genome, or its functional homolog, for example, a human herpesvirus 6 (HHV-6) Rep gene (mediating AAV-2 DNA replication is known). The Cap gene means a region in the AAV genome encoding a capsid protein of a virus known to those skilled in the art.

As the vector containing the Rep gene and the Cap gene, for example, a plasmid, a nucleic acid sequence derived from a virus, or an artificially designed nucleic acid can be used, and a plasmid is preferable.

In the present invention, a gene for treatment or prevention may be introduced into cells.

As the gene for treatment or prevention, a gene that is incomplete or lost in the genome of the target cell, a gene encoding non-natural protein having a desired biological or therapeutic effect (for example, antiviral function), or the like can be used, but the gene is not particularly limited. Specific examples of the gene for treatment or prevention include a gene used for treatment or prevention of inflammatory diseases, autoimmunity, chronic and infectious diseases (including disorders such as AIDS, cancer, nervous system diseases, cardiovascular diseases, and hypercholesterolemia), various blood diseases such as anemia and hemophilia, or gene deficiency (for example, cystic fibrosis, Gaucher's disease, adenosine deaminase (ADA) deficiency, emphysema, and the like).

The gene for treatment or prevention may be several antisense oligonucleotides (for example, a short-chain oligonucleotide complementary to a sequence around a translation initiation site (AUG codon) of mRNA) that are useful in antisense therapy against cancer and viral disease.

The gene for therapy or prophylaxis may be linked to a promoter for expressing the gene for therapy or prophylaxis. The promoter for expressing a gene for treatment or prevention is not particularly limited, but examples thereof can include a cytomegalovirus-derived promoter (including an enhancer as desired), an SV40 early promoter, a human elongation factor-1α (EF-1α) promoter, a human ubiquitin C promoter, a retrovirus Rous sarcoma virus LTR promoter, a dihydrofolate reductase promoter, a β-actin promoter, and a phosphoglycerate kinase (PGK) promoter. The gene for treatment or prevention and the promoter for expressing the gene are preferably flanked by the ITR sequences.

In the present invention, a virus helper gene derived from an adenovirus may be preferably introduced into cells. The virus helper gene is a non-adeno-associated virus gene for enabling replication and packaging of an adeno-associated virus. As the virus helper gene, a gene derived from a virus of another species other than the adeno-associated virus is used. Specific examples of the virus helper gene include a virus helper gene derived from an adenovirus or a herpesvirus, and the virus helper gene is preferably derived from an adenovirus.

Examples of the virus helper gene derived from adenovirus can include E1A, E1B, E2A, E4, and VA-RNA. In the host cell having all or a part of the E1 region, the region of the adenovirus genome required for replicating the AAV genome and packaging of the AAV genome into the capsid to form a AAV virion is the E2A region, the E4 region, and the VA1 RNA region. For the function by the E4 region, the 34 kDa E4 protein encoded by the open reading frame 6 (E4ORF6) of the E4 region is required for replicating the AAV. Preferably, the virus helper gene is an E2 gene, an E4 gene, or a VA-RNA gene. The VA-RNA gene is preferably a VA-RNAI gene.

The adenovirus-derived virus helper gene (such as an E1A, an E1B, an E2A, an E4, and a VA-RNA) may be a wild-type gene, but a gene in which modifications such as substitution, deletion, insertion, or addition of a base have been made to the wild-type gene may be used as long as it exhibits an inherent function.

In a case of introducing a virus helper gene into a cell, a vector containing the virus helper gene can be introduced into the cell.

As the vector containing a virus helper gene, for example, a plasmid, a virus-derived sequence, an artificially designed nucleic acid, or the like can be used, and a plasmid is preferable.

It is preferable that the virus helper gene is under the control of a promoter.

The specific examples of the promoter are not particularly limited, but can include a cytomegalovirus-derived promoter (CMV promoter) (including an enhancer as desired), an SV40 early promoter, a human elongation factor-1α (EF-1α) promoter, a human ubiquitin C promoter, a retrovirus Rous sarcoma virus (RSV) LTR promoter, a dihydrofolate reductase promoter, a β-actin promoter, and a phosphoglycerate kinase (PGK) promoter. The promoter may be under the control of a promoter capable of regulating expression. The promoter capable of regulating expression may be a Tet-on/off system which is a promoter capable of regulating expression by tetracycline, or a Tet-on system.

As described above, the target nucleic acid in the present invention can include a nucleic acid sequence of a gene required for the above-described virus production. For example, the target nucleic acid in the present invention can include at least one or more nucleic acid sequences of a Rep gene, a Cap gene, VA-RNA, E2, or E4.

In the present invention, a nucleic acid encoding a protein as the useful substance can also be used as the target nucleic acid. Examples of the protein include a recombinant polypeptide chain, a recombinant secreted polypeptide chain, an antigen-binding protein, an antibody (for example, a human antibody, a humanized antibody, a chimeric antibody, a mouse antibody, or a bispecific antibody), an Fc fusion protein, a fragmented immunoglobulin, and a single-chain antibody (scFv).

The protein is preferably an antibody, and more preferably a human antibody, a humanized antibody, a chimeric antibody, or a mouse antibody. Examples of the fragmented immunoglobulin include Fab, F(ab')₂, and Fv. The class of the antibody is also not particularly limited, and it may be any class of IgG such as IgG1, IgG2, IgG3, or IgG4, IgA, IgD, IgE, or IgM. However, IgG or IgM is preferable in a case of being used as a medicine.

The antibody is not particularly limited, but examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody.

In the step (b), a method of introducing the target nucleic acid into the cell is not particularly limited, but for example, the target nucleic acid can be introduced into the cell by calcium phosphate, DEAE-dextran, a cationic lipid, a polymer, a virus, magnetofection, microinjection, electroporation, or a gene gun. Among the above, it is preferable that the target nucleic acid can be introduced into the cell by a polymer, and it is more preferable that the target nucleic acid can be introduced into the cell by a cationic polymer.

The cationic polymer is not particularly limited, and for example, one type selected from the group consisting of chitosan, poly-L-lysine (pLL), polyamine (PA), polyalkyleneimine (PAI), polyethyleneimine (PEI), poly[a-(amino butyl)-L-glycolic acid], polyamidoamine, poly(2-dimethylamino)ethyl methacrylate, polyhistidine, polyarginine, poly(4-vinylpyridine), poly(vinylamine), and poly(4-vinyl-N-alkylpyridinium halide), or a combination thereof can be used.

As the cationic polymer, polyamine is preferable, and polyethyleneimine (hereinafter, also referred to as PEI) is particularly preferable. As the cationic polymer, it is preferable to use a non-lipid cationic polymer.

The PEI may be linear PEI or branched PEI, but is preferably linear PEI.

The molecular weight of PEI is preferably 10,000 to 800,000 Da, more preferably 10,000 to 50,000 Da, still more preferably 10,000 to 27,000 Da, and particularly preferably about 25,000 Da, in consideration of the transfection efficiency. As an example, linear PEI having a molecular weight of about 25,000 Da can be used. PEI is commercially available and can be easily obtained.

Transfection reagents such as DEAE-dextran, a cationic lipid, and a polymer form a complex with a nucleic acid and are taken up by cells. It is considered that the impurities (for example, cell-derived RNA) inhibit the interaction between the nucleic acid and the transfection reagent, thereby lowering the transfection efficiency. In the present invention, by removing the cause of the decrease in transfection efficiency, it is possible to proliferate the cells that take up a nucleic acid (or to obtain the same cells with a small amount of plasmid), and it is possible to produce a recombinant protein with high efficiency.

The cell used in the present invention may be any one of an isolated cell, a cell included in an isolated living body-derived tissue, or a cultured cell. In a case where the cell is an isolated cell or a cultured cell, the cell may be any of an adherent cell or a floating cell.

As the cell, an animal cell is preferable. The animal cell is not particularly limited, but is preferably a mammalian cell or an insect cell, and more preferably a mammalian cell. Examples of the mammalian cell include a human cell, a mouse cell, a rat cell, a monkey cell, and a hamster cell, but the cells are not particularly limited. The human cell can be preferably used. Examples of the cell include mouse myeloma (NSO) cells, Chinese hamster ovary (CHO) cells, HT1080, H9, HepG2, MCF7, MDBK Jurkat, NIH3T3, PC12, baby hamster kidney (BHK) cells, SP2/0, YB2/0, Y0, C127, an L cell, COS (for example, COS1 and COS7), QC1-3, human embryonic kidney cells (HEK cells) (for example, HEK293 and the like), VERO cells, PER. C6, HeLa, EB1, EB2, EB3, and oncolytic or hybridoma cell lines. The cells are preferably HEK cells, CHO cells, or Vero cells, and more preferably HEK293 cells.

The cell density of the cell-containing solution in the step (b) is preferably 1 × 10⁶ cells/mL or more, more preferably 5 × 10⁶ cells/mL or more, still more preferably 10 × 10⁶ cells/mL or more, and particularly preferably 50 × 10⁶ cells/mL or more. The upper limit is preferably 300 × 10⁶ cells/mL and more preferably 200 × 10⁶ cells/mL.

The cell density can be determined, for example, using Vi-cell (trademark) XR (manufactured by Beckman Coulter, Inc.).

The volume of the cell-containing solution is preferably 1 L or more, more preferably 10 L or more, and may be 100 L or more, 200 L or more, 500 L or more, or 1,000 L or more.

According to the present invention, there is provided a method for producing a useful substance, including a step of producing a nucleic acid-introduced cell by the method according to the embodiment of the present invention and a step of culturing the nucleic acid-introduced cell.

The useful substance is not particularly limited, and examples thereof include a virus vector and a protein. The useful substance is preferably a viral vector. The protein or virus vector, which is produced, may be used, for example, as an active pharmaceutical ingredient. The details of the virus vector or the protein are as described above in the present specification.

In the present invention, a useful substance is produced by culturing a nucleic acid-introduced cell.

The culture of cells can be performed under normal conditions for cell culture.

The culture temperature of the cells is not particularly limited, and the culture of the cells are performed at a temperature at which the cells can survive. The culture temperature is generally 25°C to 45°C, preferably 30°C to 42°C, more preferably 35°C to 40°C, and 37°C as an example.

The CO₂ concentration is generally 3 to 10% CO₂, preferably 5 to 10% CO₂, and 8% CO₂ as an example.

The period for producing a useful substance such as a virus is preferably 24 hours or more and 30 days or less, more preferably 24 hours or more and 20 days or less, still more preferably 24 hours or more and 10 days or less, even still more preferably 24 hours or more and 7 days or less, and particularly preferably 24 hours or more and 72 hours or less.

As the culture, batch culture, fed-batch culture, perfusion culture, or shaking culture can be performed. It is preferable that at least a part of the virus production step is suspension culture or perfusion culture.

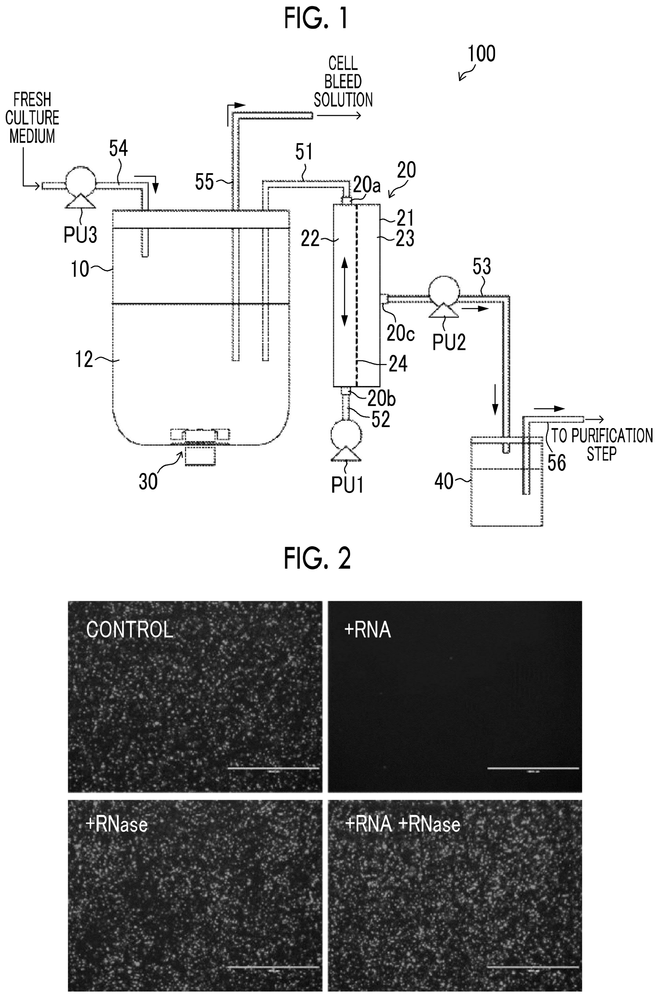

Fig. 1 is a view illustrating an example of a configuration of a cell culture device 100 according to the present invention. The cell culture device 100 includes a culture container 10 for accommodating a cell-containing solution 12 containing cells and a culture medium, a filter unit 20 having a filter membrane 24 for performing a membrane separation treatment on the cell-containing solution extracted from the culture container 10, a flow channel 51 through which the cell-containing solution that moves between the filter unit 20 and the culture container 10 passes, a flow channel 53 through which a component that has permeated through the filter membrane 24 passes, and a recovery tank 40 connected to the flow channel 53. The cell culture device 100 can be suitably used for cell culture by a perfusion culture method in which a culture medium containing a product is continuously extracted from the culture container 10, and a fresh culture medium is continuously supplied to the culture container 10. The contents described in WO2019/049843A and WO2021/187008A are incorporated in the present specification by reference.

The culture container 10 is used for large-scale culture of cells. The container 10 preferably has a capacity of 1 L or more. The capacity is more preferably 10 L or more, more preferably 100 L or more, and more preferably 1,000 L or more. The capacity is preferably 1,000,000 L or less. A stirring device 30 for stirring the cell-containing solution accommodated in the culture container 10 is provided at a bottom portion of the culture container 10. A monitoring device that monitors the amount of the unnecessary nucleic acid may be installed in the container 10. In addition, the monitoring device may be installed in the flow passages 51 and 53 or may be installed in the recovery tank 40.

The perfusion culture is a culture method in which a fresh culture medium is supplied into a cell culture solution and a part of the culture medium in which cells are cultured is removed. In a case where this perfusion culture is carried out, it is possible to remove, from a culture tank, metabolic decomposition products discharged from cells. In the perfusion culture, it is possible to recover a liquid obtained by continuously separating cells in a culture solution while continuously supplying a culture medium to a culture tank.

In general, the perfusion culture makes it possible to achieve a high viable cell density. A typical perfusion culture begins with a batch culture that continues for 1 or 2 days, thereafter a fresh supply culture medium is added to the culture continuously, stepwise, and/or intermittently, and the used culture medium is removed at the same time. In the perfusion culture, it is also possible to separate cells by using methods such as sedimentation, centrifugation, and filtration and remove the used culture medium while maintaining the viable cell density. The advantage of the perfusion culture is that the culture in which a target protein is produced is maintained for a long period as compared with the batch culture method or the fed-batch culture.

Perfusion may be any form of being continuous, stepwise, intermittent, or a combination thereof. A continuous form is preferable. Animal cells are retained in the culture and the used culture medium that is removed may substantially not include cells or may have much fewer cells than the culture. A product that is expressed by cell culture can be retained in the culture or recovered by the selection of the membrane pore diameter.

The continuous separation method for a cell culture solution in the perfusion culture is preferably a method performing continuous separation using a membrane, and is preferably membrane filtration. The membrane filtration for separating cells and a product in the culture solution is more preferably alternating tangential flow filtration (ATF method).

A method for extracting a product from a culture solution can be carried out by draining the product from the secondary side of the filtration membrane with a pump, but another available liquid feeding means may be used. The extracted culture solution is subjected to treatments such as product recovery and removal of the dead cells. In addition, the extracted culture solution may be partially discarded or returned to the culture container after treatments such as product recovery and removal of the dead cells. In a case where a loss of the culture solution occurs due to the above treatments, the loss can be compensated, for example, by supplying a fresh culture medium to the culture container.

In the perfusion culture, it is possible to adjust a cell density to be within ± 10% of the target cell density by extracting a culture solution containing cells at least once or more a day after the cell density reaches a target cell density.

Extracting a part of a culture solution together with cells so that the viable cell density during the culturing does not become excessive, thereby reducing the viable cell density is referred to as cell bleeding, and the amount of the culture solution can be maintained by adding the same amount of a fresh culture medium as the amount of the extracted culture solution. The term "once or more a day" includes a case of continuously and automatically carrying out cell bleeding.

The culture scale is not particularly limited, and the cells can be cultured in a culture medium of any volume, for example, the cells can be cultured in a culture medium of 1 mL to 2,500 L, preferably 1 L to 2,300 L, more preferably 50 L to 2,200 L, and particularly preferably 300 L to 2,100 L.

The culture may be carried out while stirring by shaking. The stirring speed in the case of stirring by shaking is generally 50 rpm to 200 rpm, and preferably 80 to 150 rpm.

The stirring culture may be rotary stirring culture using a propeller or the like in a reactor. The stirring speed in the case of stirring by rotating is generally 50 rpm to 200 rpm, and preferably 80 to 150 rpm. In addition, stirring may be performed by wave-type shaking stirring or vertical movement of the stirring blade, but the stirring is not particularly limited.

Examples of the culture medium include, but are not limited to, a Balan CD (registered trademark) culture medium, Expi293 Expression Medium (Thermo Fisher Scientific, Inc., A1435101), a Dulbecco's modified Eagle's medium (DMEM) containing 10% (vol/vol) fetal bovine serum (FBS), Gibco (trademark) Viral Production Medium (Thermo Fisher Scientific, Inc., A4817901), a serum-free UltraCULTURE (trademark) culture medium (Lonza Group AG), a HuMEC basal serum-free culture medium, a KNOCKOUT (trademark) CTS (trademark) XenoFREE ESC/iPSC culture medium, a STEMPRO (trademark)-34 SFM culture medium, a STEMPRO (trademark) NSC culture medium, an ESSENTIAL (trademark)-8 culture medium, a culture medium 254, a culture medium 106, a culture medium 131, a culture medium 154, a culture medium 171, a culture medium 200, a culture medium 231, a HeptoZYME-SFM, a human endothelial-SFM, a GIBCO (registered trademark) FREESTYLE (trademark) 293 expression culture medium, a culture medium 154CF/PRF, a culture medium 154C, a culture medium 154 CF, a culture medium 200PRF, a culture medium 131, an Essential (trademark)-6 culture medium, a STEMPRO (trademark)-34 culture medium, a Gibco (registered trademark) Astrocyte culture medium, an AIM V (registered trademark) culture medium CTS (trademark), an AMINOMAX (trademark) C-100 basal culture medium, an AMINOMAX (trademark)-II complete culture medium, a CD FORTICHO (trademark) culture medium, a CD CHO AGT culture medium, a CHO-S-SFM culture medium, a Gibco (registered trademark) FREESTYLE (trademark) CHO expression culture medium, a CD OPTICHO (trademark) culture medium, a CD CHO culture medium, a CD DG44 culture medium, a SF-900 (trademark) culture medium, an EXPI293 (trademark) expression culture medium, an LHC basal culture medium, an LHC-8 culture medium, a 293 SFM culture medium, a CD293 culture medium, an AEM growth culture medium, a PER.C6 (registered trademark) cell culture medium, an AIM V (registered trademark) culture medium, an EXPILIFE (registered trademark) culture medium, a keratinocyte SFM culture medium, an LHC culture medium, an LHC-8 culture medium, an LHC-9 culture medium, and any derivatives or modifications thereof. The culture medium may be supplemented with 1x GlutaMAX (trademark) (manufactured by Thermo Fisher Scientific, Inc.) or the like, as desired.

In a case where the useful substance produced in the present invention is a virus, the titer of the produced virus can be measured by a conventional method known to those skilled in the art. For example, the cell culture solution after the culture is collected, the cells are disrupted by freeze-thaw, and then the supernatant is collected by centrifugation. The gDNA (genomic DNA) and the residual plasmid can be digested by adding MgCl₂ and Benzonase to the collected supernatant to react. It is possible to measure the titer of a target virus by performing droplet digital PCR (ddPCR) using the sample containing the target virus obtained above as a ddPCR sample.

The present invention will be more specifically described using the following examples, but it is not limited by the examples.

### Examples

[Materials and methods]

### <Cell culture>

HEK293 cells (Thermo Fisher Scientific, Inc., A49784) were suspended in 12 mL of a culture medium for suspension culture (Balan CD HEK293: FUJIFILM Irvine Scientific, 91165-1L) and cultured in a 125 mL shaking flask. The flask was uniformly stirred at 120 rpm under the conditions of 37°C and 8% CO₂.

### <RNA preparation>

The HEK293 cell culture suspension was centrifuged at 300 × g for 5 minutes, and the supernatant was removed to recover 1 × 10⁶ cells. RNA was purified from the recovered cells using an RNeasy Micro Kit (QIAGEN, 74004). The concentration of RNA was measured using NanoDrop One (Thermo Fisher Scientific, Inc., ND-ONE-W).

### <RNase treatment of cell culture solution>

Ribonuclease A (RNase A) (QIAGEN, 19101) was added to the cell culture solution such that the final concentration thereof was 50 µg/mL. The flask was uniformly stirred at 120 rpm under the conditions of 37°C and 8% CO₂.

### <Nucleic acid detection by agarose gel electrophoresis>

After centrifugation of the HEK293 cell culture suspension at 300 × g for 5 minutes, the supernatant was recovered and centrifuged again at 10,000 × g for 5 minutes. A sample obtained by mixing 1 µL of 10× Loading Buffer (Takara Bio Inc., 9157) with 10 µL of the supernatant was subjected to electrophoresis in a 1% agarose gel containing SYBR Safe DNA Gel Stain (Thermo Fisher Scientific, Inc., S33102). The purified RNA sample prepared in the same manner was subjected to electrophoresis in an agarose gel. WSE-6100 LuminoGraphI (ATTO CORPORATION, 2006100) was used for imaging. The amount of RNA contained in the culture supernatant was calculated by measuring the band intensity ratio with the purified RNA using ImageJ.

### <Production of nucleic acid-introduced cell>

A solution obtained by adding a total amount of 21 µg of plasmid to 800 µL of Balan CD HEK293 culture medium was mixed with a solution obtained by adding 42 µL of Polyethylenimine (PEI) (PolyPlus-transfection SAS, 115-0015) to 800 µL of Balan CD HEK293 culture medium. The mixed solution was allowed to stand for 15 minutes and then added to the cell culture solution to perform transfection of the plasmid. The culture flask was uniformly stirred at 120 rpm under the conditions of 37°C and 8% CO₂. Images of the cells after the nucleic acid introduction were acquired using an EVOS FL Cell Imaging System (Thermo Fisher Scientific, Inc.).

### <Plasmid>

The used plasmids are shown below. The base sequence of the plasmid will be described later.
Plasmid 1: a plasmid in which a GFP gene under the control of a CMV promoter is inserted between two ITR sequences
Plasmid 2: a plasmid encoding a Rep gene and a Cap gene, which are derived from adeno-associated virus (Takara Bio Inc., 6230)
Plasmid 3: a plasmid encoding helper genes (E2A, E4, VA) derived from pHelper Vector adenovirus (Takara Bio Inc., 6230)

### <Measurement of GFP-expressing positive cell>

After centrifuging 0.1 mL of an HEK293 cell culture suspension at 300 × g for 5 minutes, the supernatant was removed and the cells were resuspended in 0.5 mL of PBS. The number of cells and the GFP fluorescence intensity in the cell suspension sample were measured using an Attune Flow Cytometer (Thermo Fisher Scientific, Inc.), and the expression-positive cell rate was quantitatively evaluated.

### <Protein detection by Western blot method>

Sample Buffer (nacalai tesque, 09499-14) was added to the cell culture solution and the mixture was heated at 100°C for 5 minutes to prepare a sample. The protein in the sample was size-separated using a sodium dodecyl sulfate (SDS) gel electrophoresis device (ATTO CORPORATION, 2321670), and then transferred to a membrane using a semi-dry blotting device (ATTO CORPORATION, 2322490). The membrane after the transfer was blocked with Blocking One (nacalai tesque, 03953-95) for 30 minutes, and then reacted with an anti-REP antibody (OriGene Technologies, BM5092) and an anti-β-actin antibody (Abcam, ab253283), which were diluted 70-fold with a blocking solution at room temperature for 3 hours. Next, the reaction was performed with the anti-mouse IgG-HRP antibody (Cytiva, NA931-100UL) diluted 10,000 times with a blocking solution at room temperature for 2 hours, and then the REP protein and the β-actin protein were detected by SuperSignal West Femto Maximum Sensitivity Substrate (Thermo Fisher Scientific, Inc., 34095). WSE-6100 LuminoGraphI (ATTO CORPORATION, 2006100) was used for imaging.

### [Experiment 1]

12 mL of the HEK293 cell culture solution having a cell density of 4 × 10⁶ cells/mL was treated under the following conditions, and the transfection of the plasmid 1 was performed. A fluorescence image of the cells was acquired after 24 hours from transfection (scale bar: 1,000 µm) (Fig. 2).
Control: No cell treatment before transfection
+RNase: a sample obtained by adding an RNase A solution which had been incubated at 56°C for 10 minutes such that the final concentration was 12.5 µg/mL
+RNA: a sample obtained by adding purified RNA such that the final concentration was 10 µg/mL
+RNA +RNase: a sample obtained by adding a solution obtained by mixing the purified RNA and the RNase in advance and reacting the mixture at 56°C for 10 minutes such that the final concentrations of the solution were 10 µg/mL and 12.5 µg/mL

### [Result 1]

From Fig. 2, it was suggested that the transfection efficiency was decreased because of the presence of RNA in the culture system. The decrease in the transfection efficiency by RNase was not confirmed, and it was shown that the transfection efficiency decreased by RNA could be restored by the treatment with RNase.

### [Experiment 2]

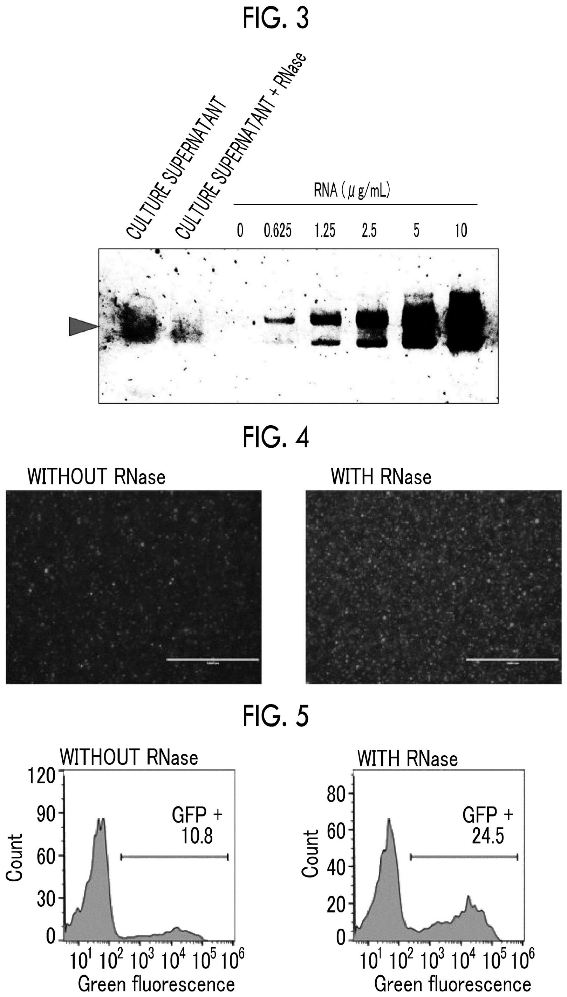

HEK293 cells were started to be cultured at 5 × 10⁵ cells/mL and proliferated to 7 × 10⁶ cells/mL, and then the culture supernatant under the condition of the presence or absence of the RNase treatment for 6 hours with respect to the cell culture solution was used as a sample. The nucleic acid contained in the culture medium was detected by agarose gel electrophoresis. The purified RNA was prepared at each concentration (0, 0.625, 1.25, 2.5, 5, and 10 µg/mL), and the nucleic acid was detected by agarose gel electrophoresis in the same manner. Fig. 3 shows the results of the agarose gel electrophoresis, and Table 1 shows the amount of nucleic acid in the sample calculated from the band intensity ratio.

### [Result 2]

From the electrophoresis results, it was shown that the cell-derived nucleic acid was accumulated in the cell culture solution. As shown in Table 1, it was shown that the amount of nucleic acid in the culture solution could be reduced by about 42% from 3.90 µg/mL to 1.63 µg/mL by the RNase treatment. In addition, it was shown that the weight ratio of RNA in the cell culture solution to the amount of plasmid (1.75 µg/mL) used for transfection was reduced from 1:2.23 to 1:0.932 by the RNase treatment.

**[Table 1]**

| | RNA concentration (µg/mL) |
|---|---|
| Culture supernatant | 3.90 |
| Culture supernatant + RNase | 1.63 |

### [Experiment 3]

In the cells under the condition of the presence or absence of the RNase treatment in Experiment 2, the plasmid 1, the plasmid 2, and the plasmid 3 were mixed in equal amounts (7 µg each), and the transfection operation was performed. A fluorescence image of the cells was acquired after 24 hours from transfection (scale bar: 1,000 µm) (Fig. 4). In addition, the quantification results of GFP-expressing positive cells using Flow cytometry are shown in Fig. 5.

### [Result 3]

The cells exhibiting fluorescence of GFP was increased from 10.8% to 24.5% by treating the cell culture solution with RNase. The reduction of the amount of nucleic acid in the culture solution contributes to the improvement of the transfection efficiency.

### [Experiment 4]

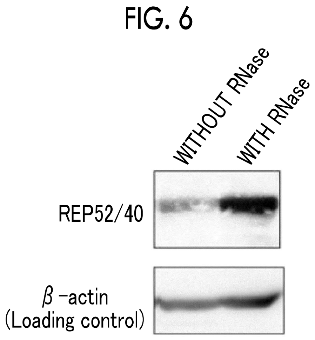

As in Experiment 3, in the cells under the condition of the presence or absence of the RNase treatment, the plasmid 1, the plasmid 2, and the plasmid 3 were mixed in equal amounts (7 µg each), and the transfection operation was performed. The cells were recovered after 72 hours from transfection, and the expression of the AAV gene Rep52/40 was detected by western blot (Fig. 6).

### [Result 4]

It was found that the expression of the REP protein required for the production of the AAV virus vector can be improved by treating the cell culture solution with RNase.

### Plasmid 1 (SEQ ID NO: 1):

GATCCACGGGTGGCATCCCTGTGACCCCTCCCCAGTGCCTCTCCTGGCCCT
GGAAGTTGCCACTCCAGTGCCCACCAGCCTTGTCCTAATAAAATTAAGTTGCATCA
TTTTGTCTGACTAGGTGTCCTTCTATAATATTATGGGGTGGAGGGGGGTGGTATGG
AGCAAGGGGCAAGTTGGGAAGACAACCTGTAGGGCCTGCGGGGTCTATTGGGAA
CCAAGCTGGAGTGCAGTGGCACAATCTTGGCTCACTGCAATCTCCGCCTCCTGGGT
TCAAGCGATTCTCCTGCCTCAGCCTCCCGAGTTGTTGGGATTCCAGGCATGCATGA
CCAGGCTCAGCTAATTTTTGTTTTTTTGGTAGAGACGGGGTTTCACCATATTGGCC
AGGCTGGTCTCCAACTCCTAATCTCAGGTGATCTACCCACCTTGGCCTCCCAAATT
GCTGGGATTACAGGCGTGAACCACTGCTCCCTTCCCTGTCCTTATCGATAGATCTA
GGAACCCCTAGTGATGGAGTTGGCCACTCCCTCTCTGCGCGCTCGCTCGCTCACTG
AGGCCGGGCGACCAAAGGTCGCCCGACGCCCGGGCTTTGCCCGGGCGGCCTCAGT
GAGCGAGCGAGCGCGCAGCTGCCTGCAGGCAGCTTGGCACTGGCCGTCGTTTTAC
AACGTCGTGACTGGGAAAACCCTGGCGTTACCCAACTTAATCGCCTTGCAGCACA
TCCCCCTTTCGCCAGCTGGCGTAATAGCGAAGAGGCCCGCACCGATCGCCCTTCCC
AACAGTTGCGCAGCCTGAATGGCGAATGGCGCCTGATGCGGTATTTTCTCCTTACG
CATCTGTGCGGTATTTCACACCGCATACGTCAAAGCAACCATAGTACGCGCCCTGT
AGCGGCGCATTAAGCGCGGCGGGTGTGGTGGTTACGCGCAGCGTGACCGCTACAC
TTGCCAGCGCCCTAGCGCCCGCTCCTTTCGCTTTCTTCCCTTCCTTTCTCGCCACGT
TCGCCGGCTTTCCCCGTCAAGCTCTAAATCGGGGGCTCCCTTTAGGGTTCCGATTT
AGTGCTTTACGGCACCTCGACCCCAAAAAACTTGATTTGGGTGATGGTTCACGTA

GTGGGCCATCGCCCTGATAGACGGTTTTTCGCCCTTTGACGTTGGAGTCCACGTTC
TTTAATAGTGGACTCTTGTTCCAAACTGGAACAACACTCAACCCTATCTCGGGCTA
TTCTTTTGATTTATAAGGGATTTTGCCGATTTCGGCCTATTGGTTAAAAAATGAGC
TGATTTAACAAAAATTTAACGCGAATTTTAACAAAATATTAACGTTTACAATTTTA
TGGTGCACTCTCAGTACAATCTGCTCTGATGCCGCATAGTTAAGCCAGCCCCGACA
CCCGCCAACACCCGCTGACGCGCCCTGACGGGCTTGTCTGCTCCCGGCATCCGCTT
ACAGACAAGCTGTGACCGTCTCCGGGAGCTGCATGTGTCAGAGGTTTTCACCGTC
ATCACCGAAACGCGCGAGACGAAAGGGCCTCGTGATACGCCTATTTTTATAGGTT
AATGTCATGATAATAATGGTTTCTTAGACGTCAGGTGGCACTTTTCGGGGAAATGT
GCGCGGAACCCCTATTTGTTTATTTTTCTAAATACATTCAAATATGTATCCGCTCAT
GAGACAATAACCCTGATAAATGCTTCAATAATATTGAAAAAGGAAGAGTATGAGT
ATTCAACATTTCCGTGTCGCCCTTATTCCCTTTTTTGCGGCATTTTGCCTTCCTGTTT
TTGCTCACCCAGAAACGCTGGTGAAAGTAAAAGATGCTGAAGATCAGTTGGGTGC
ACGAGTGGGTTACATCGAACTGGATCTCAACAGCGGTAAGATCCTTGAGAGTTTT
CGCCCCGAAGAACGTTTTCCAATGATGAGCACTTTTAAAGTTCTGCTATGTGGCGC
GGTATTATCCCGTATTGACGCCGGGCAAGAGCAACTCGGTCGCCGCATACACTAT
TCTCAGAATGACTTGGTTGAGTACTCACCAGTCACAGAAAAGCATCTTACGGATG
GCATGACAGTAAGAGAATTATGCAGTGCTGCCATAACCATGAGTGATAACACTGC
GGCCAACTTACTTCTGACAACGATCGGAGGACCGAAGGAGCTAACCGCTTTTTTG
CACAACATGGGGGATCATGTAACTCGCCTTGATCGTTGGGAACCGGAGCTGAATG
AAGCCATACCAAACGACGAGCGTGACACCACGATGCCTGTAGCAATGGCAACAA
CGTTGCGCAAACTATTAACTGGCGAACTACTTACTCTAGCTTCCCGGCAACAATTA
ATAGACTGGATGGAGGCGGATAAAGTTGCAGGACCACTTCTGCGCTCGGCCCTTC
CGGCTGGCTGGTTTATTGCTGATAAATCTGGAGCCGGTGAGCGTGGGTCTCGCGG
TATCATTGCAGCACTGGGGCCAGATGGTAAGCCCTCCCGTATCGTAGTTATCTACA
CGACGGGGAGTCAGGCAACTATGGATGAACGAAATAGACAGATCGCTGAGATAG
GTGCCTCACTGATTAAGCATTGGTAACTGTCAGACCAAGTTTACTCATATATACTT
TAGATTGATTTAAAACTTCATTTTTAATTTAAAAGGATCTAGGTGAAGATCCTTTT
TGATAATCTCATGACCAAAATCCCTTAACGTGAGTTTTCGTTCCACTGAGCGTCAG
ACCCCGTAGAAAAGATCAAAGGATCTTCTTGAGATCCTTTTTTTCTGCGCGTAATC
TGCTGCTTGCAAACAAAAAAACCACCGCTACCAGCGGTGGTTTGTTTGCCGGATC
AAGAGCTACCAACTCTTTTTCCGAAGGTAACTGGCTTCAGCAGAGCGCAGATACC
AAATACTGTTCTTCTAGTGTAGCCGTAGTTAGGCCACCACTTCAAGAACTCTGTAG

CACCGCCTACATACCTCGCTCTGCTAATCCTGTTACCAGTGGCTGCTGCCAGTGGC
GATAAGTCGTGTCTTACCGGGTTGGACTCAAGACGATAGTTACCGGATAAGGCGC
AGCGGTCGGGCTGAACGGGGGGTTCGTGCACACAGCCCAGCTTGGAGCGAACGA
CCTACACCGAACTGAGATACCTACAGCGTGAGCTATGAGAAAGCGCCACGCTTCC
CGAAGGGAGAAAGGCGGACAGGTATCCGGTAAGCGGCAGGGTCGGAACAGGAG
AGCGCACGAGGGAGCTTCCAGGGGGAAACGCCTGGTATCTTTATAGTCCTGTCGG
GTTTCGCCACCTCTGACTTGAGCGTCGATTTTTGTGATGCTCGTCAGGGGGGCGGA
GCCTATGGAAAAACGCCAGCAACGCGGCCTTTTTACGGTTCCTGGCCTTTTGCTGG
CCTTTTGCTCACATGTTCTTTCCTGCGTTATCCCCTGATTCTGTGGATAACCGTATT
ACCGCCTTTGAGTGAGCTGATACCGCTCGCCGCAGCCGAACGACCGAGCGCAGCG
AGTCAGTGAGCGAGGAAGCGGAAGAGCGCCCAATACGCAAACCGCCTCTCCCCG
CGCGTTGGCCGATTCATTAATGCAGCTGGCACGACAGGTTTCCCGACTGGAAAGC
GGGCAGTGAGCGCAACGCAATTAATGTGAGTTAGCTCACTCATTAGGCACCCCAG
GCTTTACACTTTATGCTTCCGGCTCGTATGTTGTGTGGAATTGTGAGCGGATAACA
ATTTCACACAGGAAACAGCTATGACCATGATTACGAATTGCCTGCAGGCAGCTGC
GCGCTCGCTCGCTCACTGAGGCCGCCCGGGCAAAGCCCGGGCGTCGGGCGACCTT
TGGTCGCCCGGCCTCAGTGAGCGAGCGAGCGCGCAGAGAGGGAGTGGCCAACTC
CATCACTAGGGGTTCCTATCGATATCAAGCTTTAATAGTAATCAATTACGGGGTCA
TTAGTTCATAGCCCATATATGGAGTTCCGCGTTACATAACTTACGGTAAATGGCCC
GCCTGGCTGACCGCCCAACGACCCCCGCCCATTGACGTCAATAATGACGTATGTT
CCCATAGTAACGCCAATAGGGACTTTCCATTGACGTCAATGGGTGGAGTATTTAC
GGTAAACTGCCCACTTGGCAGTACATCAAGTGTATCATATGCCAAGTACGCCCCC
TATTGACGTCAATGACGGTAAATGGCCCGCCTGGCATTATGCCCAGTACATGACC
TTATGGGACTTTCCTACTTGGCAGTACATCTACGTATTAGTCATCGCTATTACCAT
GGTGATGCGGTTTTGGCAGTACATCAATGGGCGTGGATAGCGGTTTGACTCACGG
GGATTTCCAAGTCTCCACCCCATTGACGTCAATGGGAGTTTGTTTTGGCACCAAAA
TCAACGGGACTTTCCAAAATGTCGTAACAACTCCGCCCCATTGACGCAAATGGGC
GGTAGGCGTGTACGGTGGGAGGTCTATATAAGCAGAGCTGGTTTAGTGGATATCC
TTAAGGGCCCAGCCGGCCTCGCGAGAATTCTCTAGCAAGCTGTGACCGGCGCCTA
CGCTAGACGCCACCATGGAGAGCGACGAGAGCGGCCTGCCCGCCATGGAGATCG
AGTGCCGCATCACCGGCACCCTGAACGGCGTGGAGTTCGAGCTGGTGGGCGGCGG
AGAGGGCACCCCCAAGCAGGGCCGCATGACCAACAAGATGAAGAGCACCAAAGG
CGCCCTGACCTTCAGCCCCTACCTGCTGAGCCACGTGATGGGCTACGGCTTCTACC

ACTTCGGCACCTACCCCAGCGGCTACGAGAACCCCTTCCTGCACGCCATCAACAA
CGGCGGCTACACCAACACCCGCATCGAGAAGTACGAGGACGGCGGCGTGCTGCA
CGTGAGCTTCAGCTACCGCTACGAGGCCGGCCGCGTGATCGGCGACTTCAAGGTG
GTGGGCACCGGCTTCCCCGAGGACAGCGTGATCTTCACCGACAAGATCATCCGCA
GCAACGCCACCGTGGAGCACCTGCACCCCATGGGCGATAACGTGCTGGTGGGCAG
CTTCGCCCGCACCTTCAGCCTGCGCGACGGCGGCTACTACAGCTTCGTGGTGGAC
AGCCACATGCACTTCAAGAGCGCCATCCACCCCAGCATCCTGCAGAACGGGGGCC
CCATGTTCGCCTTCCGCCGCGTGGAGGAGCTGCACAGCAACACCGAGCTGGGCAT
CGTGGAGTACCAGCACGCCTTCAAGACCCCCATCGCCTTCGCCAGATCCCGCGCT
CAGTCGTCCAATTCTGCCGTGGACGGCACCGCCGGACCCGGCTCCACCGGATCTC
GCGAGGGCAGAGGAAGTCTTCTAACATGCGGTGACGTGGAGGAGAATCCCGGCC
CTATGACCGAGTACAAGCCCACGGTGCGCCTCGCCACCCGCGACGACGTCCCCAG
GGCCGTACGCACCCTCGCCGCCGCGTTCGCCGACTACCCCGCCACGCGCCACACC
GTCGATCCGGACCGCCACATCGAGCGGGTCACCGAGCTGCAAGAACTCTTCCTCA
CGCGCGTCGGGCTCGACATCGGCAAGGTGTGGGTCGCGGACGACGGCGCCGCGGT
GGCGGTCTGGACCACGCCGGAGAGCGTCGAAGCGGGGGCGGTGTTCGCCGAGAT
CGGCCCGCGCATGGCCGAGTTGAGCGGTTCCCGGCTGGCCGCGCAGCAACAGATG
GAAGGCCTCCTGGCGCCGCACCGGCCCAAGGAGCCCGCGTGGTTCCTGGCCACCG
TCGGCGTCTCGCCCGACCACCAGGGCAAGGGTCTGGGCAGCGCCGTCGTGCTCCC
CGGAGTGGAGGCGGCCGAGCGCGCCGGGGTGCCCGCCTTCCTGGAGACCTCCGCG
CCCCGCAACCTCCCCTTCTACGAGCGGCTCGGCTTCACCGTCACCGCCGACGTCGA
GGTGCCCGAAGGACCGCGCACCTGGTGCATGACCCGCAAGCCCGGTGCCTGAAAT
CAACCTCTGGATTACAAAATTTGTGAAAGATTGACTGGTATTCTTAACTATGTTGC
TCCTTTTACGCTATGTGGATACGCTGCTTTAATGCCTTTGTATCAGTT

### Plasmid 2 (SEQ ID NO: 2):

gcgcgccgatatcgttaacgccccgcgccggccgctctagaactagtggatcccccggaagatcagaagttcctattccg
aagttcctattctctagaaagtataggaacttctgatctgcgcagccgccatgccggggttttacgagattgtgattaaggtccccagcgac
cttgacgagcatctgcccggcatttctgacagctttgtgaactgggtggccgagaaggaatgggagttgccgccagattctgacatggat
ctgaatctgattgagcaggcacccctgaccgtggccgagaagctgcagcgcgactttctgacggaatggcgccgtgtgagtaaggccc
cggaggcccttttctttgtgcaatttgagaagggagagagctacttccacatgcacgtgctcgtggaaaccaccggggtgaaatccatgg
ttttgggacgtttcctgagtcagattcgcgaaaaactgattcagagaatttaccgcgggatcgagccgactttgccaaactggttcgcggt
cacaaagaccagaaatggcgccggaggcgggaacaaggtggtggatgagtgctacatccccaattacttgctccccaaaacccagcc
tgagctccagtgggcgtggactaatatggaacagtatttaagcgcctgtttgaatctcacggagcgtaaacggttggtggcgcagcatct

gacgcacgtgtcgcagacgcaggagcagaacaaagagaatcagaatcccaattctgatgcgccggtgatcagatcaaaaacttcagc
caggtacatggagctggtcgggtggctcgtggacaaggggattacctcggagaagcagtggatccaggaggaccaggcctcatacat
ctccttcaatgcggcctccaactcgcggtcccaaatcaaggctgccttggacaatgcgggaaagattatgagcctgactaaaaccgccc
ccgactacctggtgggccagcagcccgtggaggacatttccagcaatcggatttataaaattttggaactaaacgggtacgatccccaat
atgcggcttccgtctttctgggatgggccacgaaaaagttcggcaagaggaacaccatctggctgtttgggcctgcaactaccgggaag
accaacatcgcggaggccatagcccacactgtgcccttctacgggtgcgtaaactggaccaatgagaactttcccttcaacgactgtgtc
gacaagatggtgatctggtgggaggaggggaagatgaccgccaaggtcgtggagtcggccaaagccattctcggaggaagcaaggt
gcgcgtggaccagaaatgcaagtcctcggcccagatagacccgactcccgtgatcgtcacctccaacaccaacatgtgcgccgtgatt
gacgggaactcaacgaccttcgaacaccagcagccgttgcaagaccggatgttcaaatttgaactcacccgccgtctggatcatgacttt
gggaaggtcaccaagcaggaagtcaaagactttttccggtgggcaaaggatcacgtggttgaggtggagcatgaattctacgtcaaaa
agggtggagccaagaaaagacccgcccccagtgacgcagatataagtgagcccaaacgggtgcgcgagtcagttgcgcagccatc
gacgtcagacgcggaagcttcgatcaactacgcagacaggtaccaaaacaaatgttctcgtcacgtgggcatgaatctgatgctgtttcc
ctgcagacaatgcgagagaatgaatcagaattcaaatatctgcttcactcacggacagaaagactgtttagagtgctttcccgtgtcagaa
tctcaacccgtttctgtcgtcaaaaaggcgtatcagaaactgtgctacattcatcatatcatgggaaaggtgccagacgcttgcactgcctg
cgatctggtcaatgtggatttggatgactgcatctttgaacaataaatgatttaaatcaggtatgtcttttgttgatcaccctccagattggttg
gaagaagttggtgaaggtcttcgcgagtttttgggccttgaagcgggcccaccgaaaccaaaacccaatcagcagcatcaagatcaag
cccgtggtcttgtgctgcctggttataactatctcggacccggaaacggtctcgatcgaggagagcctgtcaacagggcagacgaggtc
gcgcgagagcacgacatctcgtacaacgagcagcttgaggcgggagacaacccctacctcaagtacaaccacgcggacgccgagtt
tcaggagaagctcgccgacgacacatccttcgggggaaacctcggaaaggcagtctttcaggccaagaaaagggttctcgaacctttt
ggcctggttgaagagggtgctaagacggcccctaccggaaagcggatagacgaccactttccaaaaagaaagaaggctcggaccga
agaggactccaagccttccacctcgtcagacgccgaagctggacccagcggatcccagcagctgcaaatcccagcccaaccagcctc
aagtttgggagctgatacaatgtctgcgggaggtggcggcccattgggcgacaataaccaaggtgccgatggagtgggcaatgcctc
gggagattggcattgcgattccacgtggatgggggacagagtcgtcaccaagtccacccgaacctgggtgctgcccagctacaacaa
ccaccagtaccgagagatcaaaagcggctccgtcgacggaagcaacgccaacgcctactttggatacagcaccccctgggggtacttt
gactttaaccgcttccacagccactggagcccccgagactggcaaagactcatcaacaactactggggcttcagaccccggtccctca
gagtcaaaatcttcaacattcaagtcaaagaggtcacggtgcaggactccaccaccaccatcgccaacaacctcacctccaccgtccaa
gtgtttacggacgacgactaccagctgccctacgtcgtcggcaacgggaccgagggatgcctgccggccttccctccgcaggtctttac
gctgccgcagtacggttacgcgacgctgaaccgcgacaacacagaaaatcccaccgagaggagcagcttcttctgcctagagtacttt
cccagcaagatgctgagaacgggcaacaactttgagtttacctacaactttgaggaggtgcccttccactccagcttcgctcccagtcag
aacctgttcaagctggccaacccgctggtggaccagtacttgtaccgcttcgtgagcacaaataacactggcggagtccagttcaacaa
gaacctggccgggagatacgccaacacctacaaaaactggttcccggggcccatgggccgaacccagggctggaacctgggctccg
gggtcaaccgcgccagtgtcagcgccttcgccacgaccaataggatggagctcgagggcgcgagttaccaggtgcccccgcagccg
aacggcatgaccaacaacctccagggcagcaacacctatgccctggagaacactatgatcttcaacagccagccggcgaacccggg

caccaccgccacgtacctcgagggcaacatgctcatcaccagcgagagcgagacgcagccggtgaaccgcgtggcgtacaacgtc
ggcgggcagatggccaccaacaaccagagctccaccactgcccccgcgaccggcacgtacaacctccaggaaatcgtgcccggca
gcgtgtggatggagagggacgtgtacctccaaggacccatctgggccaagatcccagagacgggggcgcactttcacccctctccgg
ccatgggcggattcggactcaaacacccaccgcccatgatgctcatcaagaacacgcctgtgcccggaaatatcaccagcttctcgga
cgtgcccgtcagcagcttcatcacccagtacagcaccgggcaggtcaccgtggagatggagtgggagctcaagaaggaaaactcca
agaggtggaacccagagatccagtacacaaacaactacaacgacccccagtttgtggactttgccccggacagcaccggggaataca
gaaccaccagacctatcggaacccgataccttacccgacccctttaattgcttgttaatcaataaaccgtttaattcgtttcagttgaactttg
gtctctgcgtatttctttcttatctagtttccatggctacgtagataagtagcatggcgggttaatcattaactacagcccgggcgtttaaacag
cgggcggaggggtggagtcgtgacgtgaattacgtcatagggttagggaggtcctgtattagaggtcacgtgagtgttttgcgacatttt
gcgacaccatgtggtctcgctgggggggggggcccgagtgagcacgcagggtctccattttgaagcgggaggtttgaacgagcgct
ggcgcgctcactggccgtcgttttacaacgtcgtgactgggaaaaccctggcgttacccaacttaatcgccttgcagcacatccccctttc
gccagctggcgtaatagcgaagaggcccgcaccgatcgcccttcccaacagttgcgcagcctgaatggcgaatggaaattgtaagcgt
taatattttgttaaaattcgcgttaaatttttgttaaatcagctcatttttttaaccaataggccgaaatcggcaaaatcccttataaatcaaaaga
atagaccgagatagggttgagtgttgttccagtttggaacaagagtccactattaagaacgtggactccaacgtcaaagggcgaaaaac
cgtctatcagggcgatggcccactacgtgaaccatcaccctaatcaagttttttggggtcgaggtgccgtaaagcactaaatcggaaccc
taaagggagcccccgatttagagcttgacggggaaagccggcgaacgtggcgagaaaggaagggaagaaagcgaaaggagcgg
gcgctagggcgctggcaagtgtagcggtcacgctgcgcgtaaccaccacacccgccgcgcttaatgcgccgctacagggcgcgtca
ggtggcacttttcggggaaatgtgcgcggaacccctatttgtttatttttctaaatacattcaaatatgtatccgctcatgagacaataaccct
gataaatgcttcaataatattgaaaaaggaagagtatgagtattcaacatttccgtgtcgcccttattcccttttttgcggcattttgccttcctgt
ttttgctcacccagaaacgctggtgaaagtaaaagatgctgaagatcagttgggtgcacgagtgggttacatcgaactggatctcaacag
cggtaagatccttgagagttttcgccccgaagaacgttttccaatgatgagcacttttaaagttctgctatgtggcgcggtattatcccgtatt
gacgccgggcaagagcaactcggtcgccgcatacactattctcagaatgacttggttgagtactcaccagtcacagaaaagcatcttac
ggatggcatgacagtaagagaattatgcagtgctgccataaccatgagtgataacactgcggccaacttacttctgacaacgatcggag
gaccgaaggagctaaccgcttttttgcacaacatgggggatcatgtaactcgccttgatcgttgggaaccggagctgaatgaagccatac
caaacgacgagcgtgacaccacgatgcctgtagcaatggcaacaacgttgcgcaaactattaactggcgaactacttactctagcttccc
ggcaacaattaatagactggatggaggcggataaagttgcaggaccacttctgcgctcggcccttccggctggctggtttattgctgata
aatctggagccggtgagcgtgggtctcgcggtatcattgcagcactggggccagatggtaagccctcccgtatcgtagttatctacacga
cggggagtcaggcaactatggatgaacgaaatagacagatcgctgagataggtgcctcactgattaagcattggtaactgtcagaccaa
gtttactcatatatactttagattgatttaaaacttcatttttaatttaaaaggatctaggtgaagatcctttttgataatctcatgaccaaaatccct
taacgtgagttttcgttccactgagcgtcagaccccgtagaaaagatcaaaggatcttcttgagatcctttttttctgcgcgtaatctgctgctt
gcaaacaaaaaaaccaccgctaccagcggtggtttgtttgccggatcaagagctaccaactctttttccgaaggtaactggcttcagcag
agcgcagataccaaatactgttcttctagtgtagccgtagttaggccaccacttcaagaactctgtagcaccgcctacatacctcgctctgc
taatcctgttaccagtggctgctgccagtggcgataagtcgtgtcttaccgggttggactcaagacgatagttaccggataaggcgcagc

ggtcgggctgaacggggggttcgtgcacacagcccagcttggagcgaacgacctacaccgaactgagatacctacagcgtgagctat
gagaaagcgccacgcttcccgaagggagaaaggcggacaggtatccggtaagcggcagggtcggaacaggagagcgcacgagg
gagcttccagggggaaacgcctggtatctttatagtcctgtcgggtttcgccacctctgacttgagcgtcgatttttgtgatgctcgtcagg
ggggcggagcctatggaaaaacgccagcaacgcggcctttttacggttcctggccttttgctggccttttgctcacatgttctttcctgcgtt
atcccctgattctgtggataaccgtattaccgcctttgagtgagctgataccgctcgccgcagccgaacgaccgagcgcagcgagtcag
tgagcgaggaagcggaagagcgcccaatacgcaaaccgcctctccccgcgcgttggccgattcattaatgcagctggcacgacaggt
ttcccgactggaaagcgggcagtgagcgcaacgcaattaatgtgagttagctcactcattaggcaccccaggctttacactttatgcttcc
ggctcgtatgttgtgtggaattgtgagcggataacaatttcacacaggaaacagctatgaccatgattacgccaa

### Plasmid 3 (SEQ ID NO: 3):

ggtacccaactccatgcttaacagtccccaggtacagcccaccctgcgtcgcaaccaggaacagctctacagcttcctgga
gcgccactcgccctacttccgcagccacagtgcgcagattaggagcgccacttctttttgtcacttgaaaaacatgtaaaaataatgtacta
ggagacactttcaataaaggcaaatgtttttatttgtacactctcgggtgattatttaccccccacccttgccgtctgcgccgtttaaaaatca
aaggggttctgccgcgcatcgctatgcgccactggcagggacacgttgcgatactggtgtttagtgctccacttaaactcaggcacaacc
atccgcggcagctcggtgaagttttcactccacaggctgcgcaccatcaccaacgcgtttagcaggtcgggcgccgatatcttgaagtc
gcagttggggcctccgccctgcgcgcgcgagttgcgatacacagggttgcagcactggaacactatcagcgccgggtggtgcacgct
ggccagcacgctcttgtcggagatcagatccgcgtccaggtcctccgcgttgctcagggcgaacggagtcaactttggtagctgccttc
ccaaaaagggtgcatgcccaggctttgagttgcactcgcaccgtagtggcatcagaaggtgaccgtgcccggtctgggcgttaggata
cagcgcctgcatgaaagccttgatctgcttaaaagccacctgagcctttgcgccttcagagaagaacatgccgcaagacttgccggaaa
actgattggccggacaggccgcgtcatgcacgcagcaccttgcgtcggtgttggagatctgcaccacatttcggccccaccggttcttca
cgatcttggccttgctagactgctccttcagcgcgcgctgcccgttttcgctcgtcacatccatttcaatcacgtgctccttatttatcataatg
ctcccgtgtagacacttaagctcgccttcgatctcagcgcagcggtgcagccacaacgcgcagcccgtgggctcgtggtgcttgtaggt
tacctctgcaaacgactgcaggtacgcctgcaggaatcgccccatcatcgtcacaaaggtcttgttgctggtgaaggtcagctgcaaccc
gcggtgctcctcgtttagccaggtcttgcatacggccgccagagcttccacttggtcaggcagtagcttgaagtttgcctttagatcgttatc
cacgtggtacttgtccatcaacgcgcgcgcagcctccatgcccttctcccacgcagacacgatcggcaggctcagcgggtttatcaccg
tgctttcactttccgcttcactggactcttccttttcctcttgcgtccgcataccccgcgccactgggtcgtcttcattcagccgccgcaccgt
gcgcttacctcccttgccgtgcttgattagcaccggtgggttgctgaaacccaccatttgtagcgccacatcttctctttcttcctcgctgtcc
acgatcacctctggggatggcgggcgctcgggcttgggagaggggcgcttctttttctttttggacgcaatggccaaatccgccgtcga
ggtcgatggccgcgggctgggtgtgcgcggcaccagcgcatcttgtgacgagtcttcttcgtcctcggactcgagacgccgcctcagc
cgcttttttgggggcgcgcggggaggcggcggcgacggcgacggggacgacacgtcctccatggttggtggacgtcgcgccgcac
cgcgtccgcgctcgggggtggtttcgcgctgctcctcttcccgactggccatttccttctcctataggcagaaaaagatcatggagtcagt
cgagaaggaggacagcctaaccgccccctttgagttcgccaccaccgcctccaccgatgccgccaacgcgcctaccaccttccccgt
cgaggcacccccgcttgaggaggaggaagtgattatcgagcaggacccaggttttgtaagcgaagacgacgaggatcgctcagtacc
aacagaggataaaaagcaagaccaggacgacgcagaggcaaacgaggaacaagtcgggcggggggaccaaaggcatggcgact

acctagatgtgggagacgacgtgctgttgaagcatctgcagcgccagtgcgccattatctgcgacgcgttgcaagagcgcagcgatgt
gcccctcgccatagcggatgtcagccttgcctacgaacgccacctgttctcaccgcgcgtaccccccaaacgccaagaaaacggcac
atgcgagcccaacccgcgcctcaacttctaccccgtatttgccgtgccagaggtgcttgccacctatcacatctttttccaaaactgcaag
atacccctatcctgccgtgccaaccgcagccgagcggacaagcagctggccttgcggcagggcgctgtcatacctgatatcgcctcgc
tcgacgaagtgccaaaaatctttgagggtcttggacgcgacgagaaacgcgcggcaaacgctctgcaacaagaaaacagcgaaaatg
aaagtcactgtggagtgctggtggaacttgagggtgacaacgcgcgcctagccgtgctgaaacgcagcatcgaggtcacccactttgc
ctacccggcacttaacctaccccccaaggttatgagcacagtcatgagcgagctgatcgtgcgccgtgcacgacccctggagagggat
gcaaacttgcaagaacaaaccgaggagggcctacccgcagttggcgatgagcagctggcgcgctggcttgagacgcgcgagcctgc
cgacttggaggagcgacgcaagctaatgatggccgcagtgcttgttaccgtggagcttgagtgcatgcagcggttctttgctgacccgg
agatgcagcgcaagctagaggaaacgttgcactacacctttcgccagggctacgtgcgccaggcctgcaaaatttccaacgtggagct
ctgcaacctggtctcctaccttggaattttgcacgaaaaccgcctcgggcaaaacgtgcttcattccacgctcaagggcgaggcgcgcc
gcgactacgtccgcgactgcgtttacttatttctgtgctacacctggcaaacggccatgggcgtgtggcagcaatgcctggaggagcgc
aacctaaaggagctgcagaagctgctaaagcaaaacttgaaggacctatggacggccttcaacgagcgctccgtggccgcgcacctg
gcggacattatcttccccgaacgcctgcttaaaaccctgcaacagggtctgccagacttcaccagtcaaagcatgttgcaaaactttagg
aactttatcctagagcgttcaggaattctgcccgccacctgctgtgcgcttcctagcgactttgtgcccattaagtaccgtgaatgccctcc
gccgctttggggtcactgctaccttctgcagctagccaactaccttgcctaccactccgacatcatggaagacgtgagcggtgacggcct
actggagtgtcactgtcgctgcaacctatgcaccccgcaccgctccctggtctgcaattcgcaactgcttagcgaaagtcaaattatcggt
acctttgagctgcagggtccctcgcctgacgaaaagtccgcggctccggggttgaaactcactccggggctgtggacgtcggcttacct
tcgcaaatttgtacctgaggactaccacgcccacgagattaggttctacgaagaccaatcccgcccgccaaatgcggagcttaccgcct
gcgtcattacccagggccacatccttggccaattgcaagccatcaacaaagcccgccaagagtttctgctacgaaagggacggggggt
ttacctggacccccagtccggcgaggagctcaacccaatccccccgccgccgcagccctatcagcagccgcgggcccttgcttccca
ggatggcacccaaaaagaagctgcagctgccgccgccgccacccacggacgaggaggaatactgggacagtcaggcagaggagg
ttttggacgaggaggaggagatgatggaagactgggacagcctagacgaagcttccgaggccgaagaggtgtcagacgaaacaccg
tcaccctcggtcgcattcccctcgccggcgccccagaaattggcaaccgttcccagcatcgctacaacctccgctcctcaggcgccgcc
ggcactgcctgttcgccgacccaaccgtagatgggacaccactggaaccagggccggtaagtctaagcagccgccgccgttagccca
agagcaacaacagcgccaaggctaccgctcgtggcgcgggcacaagaacgccatagttgcttgcttgcaagactgtgggggcaacat
ctccttcgcccgccgctttcttctctaccatcacggcgtggccttcccccgtaacatcctgcattactaccgtcatctctacagcccctactg
caccggcggcagcggcagcggcagcaacagcagcggtcacacagaagcaaaggcgaccggatagcaagactctgacaaagccc
aagaaatccacagcggcggcagcagcaggaggaggagcgctgcgtctggcgcccaacgaacccgtatcgacccgcgagcttagaa
ataggatttttcccactctgtatgctatatttcaacaaagcaggggccaagaacaagagctgaaaataaaaaacaggtctctgcgctccct
cacccgcagctgcctgtatcacaaaagcgaagatcagcttcggcgcacgctggaagacgcggaggctctcttcagcaaatactgcgc
gctgactcttaaggactagtttcgcgccctttctcaaatttaagcgcgaaaactacgtcatctccagcggccacacccggcgccagcacc
tgtcgtcagcgccattatgagcaaggaaattcccacgccctacatgtggagttaccagccacaaatgggacttgcggctggagctgccc

aagactactcaacccgaataaactacatgagcgcgggaccccacatgatatcccgggtcaacggaatccgcgcccaccgaaaccgaa
ttctcctcgaacaggcggctattaccaccacacctcgtaataaccttaatccccgtagttggcccgctgccctggtgtaccaggaaagtcc
cgctcccaccactgtggtacttcccagagacgcccaggccgaagttcagatgactaactcaggggcgcagcttgcgggcggctttcgt
cacagggtgcggtcgcccgggcgttttagggcggagtaacttgcatgtattgggaattgtagtttttttaaaatgggaagtgacgtatcgtg
ggaaaacggaagtgaagatttgaggaagttgtgggttttttggctttcgtttctgggcgtaggttcgcgtgcggttttctgggtgttttttgtg
gactttaaccgttacgtcattttttagtcctatatatactcgctctgtacttggccctttttacactgtgactgattgagctggtgccgtgtcgagt
ggtgttttttaataggtttttttactggtaaggctgactgttatggctgccgctgtggaagcgctgtatgttgttctggagcgggagggtgcta
ttttgcctaggcaggagggtttttcaggtgtttatgtgtttttctctcctattaattttgttatacctcctatgggggctgtaatgttgtctctacgcc
tgcgggtatgtattcccccgggctatttcggtcgctttttagcactgaccgatgttaaccaacctgatgtgtttaccgagtcttacattatgact
ccggacatgaccgaggaactgtcggtggtgctttttaatcacggtgaccagtttttttacggtcacgccggcatggccgtagtccgtcttat
gcttataagggttgtttttcctgttgtaagacaggcttctaatgtttaaatgtttttttttttgttattttattttgtgtttaatgcaggaacccgcagac
atgtttgagagaaaaatggtgtctttttctgtggtggttccggaacttacctgcctttatctgcatgagcatgactacgatgtgcttgcttttttg
cgcgaggctttgcctgattttttgagcagcaccttgcattttatatcgccgcccatgcaacaagcttacataggggctacgctggttagcata
gctccgagtatgcgtgtcataatcagtgtgggttcttttgtcatggttcctggcggggaagtggccgcgctggtccgtgcagacctgcac
gattatgttcagctggccctgcgaagggacctacgggatcgcggtatttttgttaatgttccgcttttgaatcttatacaggtctgtgaggaa
cctgaatttttgcaatcatgattcgctgcttgaggctgaaggtggagggcgctctggagcagatttttacaatggccggacttaatattcgg
gatttgcttagagacatattgataaggtggcgagatgaaaattatttgggcatggttgaaggtgctggaatgtttatagaggagattcaccc
tgaagggtttagcctttacgtccacttggacgtgagggcagtttgccttttggaagccattgtgcaacatcttacaaatgccattatctgttctt
tggctgtagagtttgaccacgccaccggaggggagcgcgttcacttaatagatcttcattttgaggttttggataatcttttggaataaaaaa
aaaaaaacatggttcttccagctcttcccgctcctcccgtgtgtgactcgcagaacgaatgtgtaggttggctgggtgtggcttattctgcg
gtggtggatgttatcagggcagcggcgcatgaaggagtttacatagaacccgaagccagggggcgcctggatgctttgagagagtgg
atatactacaactactacacagagcgagctaagcgacgagaccggagacgcagatctgtttgtcacgcccgcacctggttttgcttcagg
aaatatgactacgtccggcgttccatttggcatgacactacgaccaacacgatctcggttgtctcggcgcactccgtacagtagggatcg
cctacctccttttgagacagagacccgcgctaccatactggaggatcatccgctgctgcccgaatgtaacactttgacaatgcacaacgt
gagttacgtgcgaggtcttccctgcagtgtgggatttacgctgattcaggaatgggttgttccctgggatatggttctgacgcgggaggag
cttgtaatcctgaggaagtgtatgcacgtgtgcctgtgttgtgccaacattgatatcatgacgagcatgatgatccatggttacgagtcctg
ggctctccactgtcattgttccagtcccggttccctgcagtgcatagccggcgggcaggttttggccagctggtttaggatggtggtggat
ggcgccatgtttaatcagaggtttatatggtaccgggaggtggtgaattacaacatgccaaaagaggtaatgtttatgtccagcgtgtttat
gaggggtcgccacttaatctacctgcgcttgtggtatgatggccacgtgggttctgtggtccccgccatgagctttggatacagcgccttg
cactgtgggattttgaacaatattgtggtgctgtgctgcagttactgtgctgatttaagtgagatcagggtgcgctgctgtgcccggaggac
aaggcgtctcatgctgcgggcggtgcgaatcatcgctgaggagaccactgccatgttgtattcctgcaggacggagcggcggcggca
gcagtttattcgcgcgctgctgcagcaccaccgccctatcctgatgcacgattatgactctacccccatgtaggcgtggacttccccttcg
ccgcccgttgagcaaccgcaagttggacagcagcctgtggctcagcagctggacagcgacatgaacttaagcgagctgcccgggga

gtttattaatatcactgatgagcgtttggctcgacaggaaaccgtgtggaatataacacctaagaatatgtctgttacccatgatatgatgctt
tttaaggccagccggggagaaaggactgtgtactctgtgtgttgggagggaggtggcaggttgaatactagggttctgtgagtttgatta
aggtacggtgatcaatataagctatgtggtggtggggctatactactgaatgaaaaatgacttgaaattttctgcaattgaaaaataaacac
gttgaaacataacatgcaacaggttcacgattctttattcctgggcaatgtaggagaaggtgtaagagttggtagcaaaagtttcagtggtg
tattttccactttcccaggaccatgtaaaagacatagagtaagtgcttacctcgctagtttctgtggattcactagaatcgatgtaggatgttg
cccctcctgacgcggtaggagaaggggagggtgccctgcatgtctgccgctgctcttgctcttgccgctgctgaggaggggggcgcat
ctgccgcagcaccggatgcatctgggaaaagcaaaaaaggggctcgtccctgtttccggaggaatttgcaagcggggtcttgcatgac
ggggaggcaaacccccgttcgccgcagtccggccggcccgagactcgaaccgggggtcctgcgactcaacccttggaaaataaccc
tccggctacagggagcgagccacttaatgctttcgctttccagcctaaccgcttacgccgcgcgcggccagtggccaaaaaagctagc
gcagcagccgccgcgcctggaaggaagccaaaaggagcgctcccccgttgtctgacgtcgcacacctgggttcgacacgcgggcg
gtaaccgcatggatcacggcggacggccggatccggggttcgaaccccggtcgtccgccatgatacccttgcgaatttatccaccaga
ccacggaagagtgcccgcttacaggctctccttttgcacggtctagagcgtcaacgactgcgcacgcctcaccggccagagcgtcccg
accatggagcactttttgccgctgcgcaacatctggaaccgcgtccgcgactttccgcgcgcctccaccaccgccgccggcatcacctg
gatgtccaggtacatctacggattacgtcgacgtttaaaccatatgatcagctcactcaaaggcggtaatacggttatccacagaatcagg
ggataacgcaggaaagaacatgtgagcaaaaggccagcaaaaggccaggaaccgtaaaaaggccgcgttgctggcgtttttccatag
gctccgcccccctgacgagcatcacaaaaatcgacgctcaagtcagaggtggcgaaacccgacaggactataaagataccaggcgtt
tccccctggaagctccctcgtgcgctctcctgttccgaccctgccgcttaccggatacctgtccgcctttctcccttcgggaagcgtggcg
ctttctcatagctcacgctgtaggtatctcagttcggtgtaggtcgttcgctccaagctgggctgtgtgcacgaaccccccgttcagcccga
ccgctgcgccttatccggtaactatcgtcttgagtccaacccggtaagacacgacttatcgccactggcagcagccactggtaacaggat
tagcagagcgaggtatgtaggcggtgctacagagttcttgaagtggtggcctaactacggctacactagaagaacagtatttggtatctg
cgctctgctgaagccagttaccttcggaaaaagagttggtagctcttgatccggcaaacaaaccaccgctggtagcggtggtttttttgttt
gcaagcagcagattacgcgcagaaaaaaaggatctcaagaagatcctttgatcttttctacggggtctgacgctcagtggaacgaaaact
cacgttaagggattttggtcatgagattatcaaaaaggatcttcacctagatccttttaaattaaaaatgaagttttaaatcaatctaaagtatat
atgagtaaacttggtctgacagttaccaatgcttaatcagtgaggcacctatctcagcgatctgtctatttcgttcatccatagttgcctgactc
cccgtcgtgtagataactacgatacgggagggcttaccatctggccccagtgctgcaatgataccgcgagacccacgctcaccggctc
cagatttatcagcaataaaccagccagccggaagggccgagcgcagaagtggtcctgcaactttatccgcctccatccagtctattaatt
gttgccgggaagctagagtaagtagttcgccagttaatagtttgcgcaacgttgttgccattgctacaggcatcgtggtgtcacgctcgtc
gtttggtatggcttcattcagctccggttcccaacgatcaaggcgagttacatgatcccccatgttgtgcaaaaaagcggttagctccttcg
gtcctccgatcgttgtcagaagtaagttggccgcagtgttatcactcatggttatggcagcactgcataattctcttactgtcatgccatccgt
aagatgcttttctgtgactggtgagtactcaaccaagtcattctgagaatagtgtatgcggcgaccgagttgctcttgcccggcgtcaatac
gggataataccgcgccacatagcagaactttaaaagtgctcatcattggaaaacgttcttcggggcgaaaactctcaaggatcttaccgct
gttgagatccagttcgatgtaacccactcgtgcacccaactgatcttcagcatcttttactttcaccagcgtttctgggtgagcaaaaacagg
aaggcaaaatgccgcaaaaaagggaataagggcgacacggaaatgttgaatactcatactcttcctttttcaatattattgaagcatttatc

agggttattgtctcatgagcggatacatatttgaatgtatttagaaaaataaacaaataggggttccgcgcacatttccccgaaaagtgcca
cctaaattgtaagcgttaatattttgttaaaattcgcgttaaatttttgttaaatcagctcattttttaaccaataggccgaaatcggcaaaatccc
ttataaatcaaaagaatagaccgagatagggttgagtgttgttccagtttggaacaagagtccactattaaagaacgtggactccaacgtc
aaagggcgaaaaaccgtctatcagggcgatggcccactacgtgaaccatcaccctaatcaagttttttggggtcgaggtgccgtaaagc
actaaatcggaaccctaaagggagcccccgatttagagcttgacggggaaagccggcgaacgtggcgagaaaggaagggaagaaa
gcgaaaggagcgggcgctagggcgctggcaagtgtagcggtcacgctgcgcgtaaccaccacacccgccgcgcttaatgcgccgct
acagggcgcgatggatcc

### Explanation of References

10: culture container
12: cell-containing solution
20: filter unit
20a: flow port
20b: flow port
20c: discharge port
21: container
22: supply side
23: permeation side
24: filter membrane
30: stirring device
40: recovery tank
51: flow channel
52: flow channel
53: flow channel
54: flow channel
55: flow channel
56: flow channel
100: cell culture device
PU1: pump
PU2: pump
PU3: pump
[Sequence list]
International application 22F01532W1JP24000886_1.xml based on International Patent Cooperation Treaty

## Claims

1. A method for producing a nucleic acid-introduced cell, comprising:
(a) a treatment step of reducing an unnecessary nucleic acid in a cell-containing solution; and
(b) an introduction step of introducing a target nucleic acid into a cell by adding the target nucleic acid to the cell-containing solution.

2. The method according to claim 1,
wherein the unnecessary nucleic acid is RNA.

3. The method according to claim 1 or 2,
wherein the treatment step includes adding an RNase to the cell-containing solution.

4. The method according to claim 1 or 2,
wherein in the step (a), an amount of the unnecessary nucleic acid is monitored and reduced to an amount equal to or less than a reference value.

5. The method according to claim 1 or 2,
wherein the target nucleic acid is a purified nucleic acid.

6. The method according to claim 1 or 2,
wherein the step (b) is performed after the step (a) is performed.

7. The method according to claim 1 or 2,
wherein the target nucleic acid is DNA.

8. The method according to claim 1 or 2,
wherein the target nucleic acid is a plasmid.

9. The method according to claim 1 or 2,
wherein, in the step (b), the target nucleic acid is introduced into the cell by calcium phosphate, DEAE-dextran, a cationic lipid, a polymer, a virus, magnetofection, microinjection, electroporation, or a gene gun.

10. The method according to claim 1 or 2,
wherein, in the step (b), the target nucleic acid is introduced into the cell by a polymer.

11. The method according to claim 10,
wherein the polymer is polyethyleneimine.

12. The method according to claim 1 or 2,
wherein the cell is an HEK cell, a CHO cell, or a Vero cell.

13. The method according to claim 1 or 2,
wherein a cell density of the cell-containing solution in the step (b) is 1 × 10⁶ cells/mL or more.

14. The method according to claim 1 or 2,
wherein a volume of the cell-containing solution is 1 L or more.

15. A method for producing a useful substance, comprising:
a step of producing a nucleic acid-introduced cell by the method according to claim 1 or 2; and
a step of culturing the nucleic acid-introduced cell.

16. The method according to claim 15,
wherein the useful substance is a virus vector or a protein.
